# EUROPÄISCHE PATENTSCHRIFT

(11) **EP 2 914 616 B1**
(45) Veröffentlichungstag und Bekanntmachung des Hinweises auf die Patenterteilung: **03.03.2021**
(21) Anmeldenummer: 13786411.2
(22) Anmeldetag: 25.09.2013
(51) Int. Cl.: C07K 7/08, G01N 33/68, A61P 25/28, A61K 38/10

(54) **PEPTIDE, DIE AN AMINO-TERMINAL VERKÜRZTES AMYLOID-BETA-PEPTID BINDEN UND DEREN VERWENDUNG**
PEPTIDES THAT BIND TO AMINO-TERMINAL TRUNCATED AMYLOID-BETA-PEPTIDE AND USE OF SAID PEPTIDES
PEPTIDES SE LIANT AU PEPTIDE AMYLOÏDE BÊTA RACCOURCI DANS LA PARTIE N-TERMINALE ET LEUR UTILISATION

(30) Priorität: 02.11.2012 DE 102012022013
(43) Veröffentlichungstag der Anmeldung: 09.09.2015
(73) Patentinhaber: Forschungszentrum Jülich GmbH, 52425 Jülich (DE)
(72) Erfinder: WILLBOLD, Dieter, 52425 Jülich (DE); FUNKE, Susanne, Aileen, 96242 Sonnefeld (DE); CINAR, Yeliz, 65203 Wiesbaden (DE); BARTNIK, Dirk, 41334 Nettetal (DE); DEMUTH, Hans-Ulrich, 06120 Halle / Saale (DE); KLEINSCHMIDT, Martin, 06114 Halle / Saale (DE); LUDWIG, Hans-Henning, 06110 Halle / Saale (DE)
(86) Internationale Anmeldenummer: PCT/DE2013/000543
(87) Internationale Veröffentlichungsnummer: WO 2014/067505

(56) Entgegenhaltungen:
- EP-B1- 1 379 546
- WO-A1-2012/136552
- KATJA WIESEHAN ET AL: "Selection of D-Amino-Acid Peptides That Bind to Alzheimer's Disease Amyloid Peptide A[beta]142 by Mirror Image Phage Display", CHEMBIOCHEM, Bd. 4, Nr. 8, 4. August 2003 (2003-08-04), Seiten 748-753, XP055069247, ISSN: 1439-4227, DOI: 10.1002/cbic.200300631

### SEQUENCE LISTING

<110> Forschungszentrum Jülich GmbH
<120> Peptide, die an amino-terminal verkürztes Amyloid-beta-Peptid binden und deren Verwendung
<130> PT 1.2595
<160> 4
<170> PatentIn version 3.5
<210> 1
   <211> 12
   <212> PRT
   <213> Artificial Sequence
<220>
   <223> D7 - Peptid
<400> 1
   His Thr Arg Phe Glu Tyr Tyr Val Tyr His Met Ser
   1               5                   10
<210> 2
   <211> 12
   <212> PRT
   <213> Artificial Sequence
<220>
   <223> D6 - Peptid
<400> 2
   Ala Gly Glu Arg Leu Lys Phe Ile Asp Glu His Val
   1               5                   10
<210> 3
   <211> 12
   <212> PRT
   <213> Artificial Sequence
<220>
   <223> D4 - Peptid
<400> 3
   Lys Met Glu His Pro Asn His Pro Pro Pro Gln Arg
   1               5                   10
<210> 4
   <211> 12
   <212> PRT
   <213> Artificial Sequence
<220>
   <223> D5 - Peptid
<400> 4
   Asn Gly Ala Pro Asn Lys Ile Pro Arg Asp Arg Glu
   1               5                   10

## Beschreibung

Die Erfindung bezieht sich auf Peptide, die an amino-terminal verkürztes Amyloid-beta-Peptid (AβpE3-x) binden, Sonden enthaltend diese Peptide sowie Verfahren zum Nachweis von Plaques, welche zumindest teilweise aus amino-terminal verkürzten Amyloid-beta-Peptiden bestehen.

### Stand der Technik

Die Alzheimersche Demenz (AD) ist die häufigste Demenzform und betrifft ca. 20 Millionen Menschen weltweit. Pathologisches Hauptmerkmal der AD ist die Bildung von senilen oder amyloiden Plaques, bestehend aus dem Aβ-Peptid (Amyloid-Beta-Peptid, A-Beta-Peptid), und neurofibrillären Ablagerungen aus dem Tau-Protein. Die Amyloid-Kaskadenhypothese entstand in den 90er Jahren und postuliert, dass die Ablagerung von Aβ in Form von Plaques Auslöser der Krankheitssymptome ist. Neuere Studien weisen darauf hin, dass kleinere, frei diffundierbare Aβ-Oligomere toxischer sind als die in den Plaques abgelagerten Aβ-Fibrillen. Neuen Arbeiten zufolge können die Plaques als Reservoir für oligomeres Aβ angesehen werden, welches mit der Zerstörung von Synapsen und Neuronen kolokalisiert. Das Aβ-Peptid entsteht durch die Aktivitäten mindestens zweier verschiedener Proteasen aus einem Vorläuferprotein, dem "Amyloid Precursor Protein" (APP). Dieses ist in der Zellwand von Neuronen lokalisiert. Bei dem proteolytischen Abbau von APP und durch nachträgliche Modifikation entstehen Aβ-Fragmente unterschiedlicher Länge und Art. Nach Verdau durch die □gamma-Sekretase werden Aβ-Peptide unterschiedlicher Länge gebildet, z. B. Aβ 1-42, Aβ 1-40 und so weiter. Diese Spezies unterscheiden sich in ihrer Tendenz zur Aggregation. Zusätzlich ist bekannt, dass ein Teil der im menschlichen Gehirn vorkommenden Aβ-Spezies nicht mehr als ursprüngliches Peptid nachgewiesen werden kann, sondern als trunkiertes AβpE3-x. AβpE3-x sind amino-terminal verkürzte Peptide, deren freiliegender Glutaminsäure-Rest an der Position 3 in zyklisierter Pyroglutamatform vorliegt. Der C-Terminus ist variabel, z. B. AβpE3-40, AβpE3-42 und so weiter. AβpE3-x, vor allem AβpE3-42, wird zu einem großen Anteil im Zentrum der amyloiden Plaques detektiert und ist deutlich aggregationsfreudiger und zelltoxischer als nicht amino-terminal trunkiertes und modifiziertes Aβ.

Es werden daher Substanzen benötigt, die die Menge von toxischen Aβ Oligomeren und/oder trunkierten und veränderten Aβ-Spezies reduzieren.

Es existiert bisher kein ursächlich wirkendes Medikament gegen die Alzheimersche Demenz. Die bisher eingesetzten Medikamente sind bestenfalls in der Lage, einige Symptome zu mildern, können aber den Krankheitsfortschritt nicht verlangsamen, geschweige denn aufhalten.

Nachteilig können somit bisher nur die Symptome der Alzheimerschen Demenz behandelt werden. Es gibt keine zugelassenen Medikamente, die die Krankheitsprozesse aufhalten oder rückgängig machen können. Die meisten der Substanzen, die für die Therapie der Alzheimerschen Demenz erforscht werden, fokussieren auf extrazelluläres Aß, dabei aber nicht gezielt auf lösliche Aß-Oligomere oder aggregationsfreudige trunkierte Aß-Spezies wie AßpE3-42. Um den Krankheitsprozess in frühen Stadien aufhalten zu können, ist aber genau das nötig.

Des Weiteren gibt es bisher keine Möglichkeit, die Alzheimersche Demenz vor dem Ausbruch der Symptome zu diagnostizieren. Die Alzheimersche Demenz wird heute hauptsächlich durch neuropsychologische Tests der bereits an Demenzsymptomen leidenden Person erkannt. Des Weiteren können andere Erkrankungen (Traumata) durch verschiedene Untersuchungsmethoden ausgeschlossen werden. Es ist jedoch bekannt, dass Aß-Oligomere und zeitlich darauf folgend Plaques bis zu 20 Jahre vor dem Auftreten der Symptome im Gehirn der Patienten entstehen und irreversiblen Schaden anrichten. Aus der WO2012/136552 sind beispielsweise Antikörper, die an pGlu-Aβ(3-40/42) binden bekannt, die in der Diagnose und Therapie von Alzheimer-Demenz verwendet werden. In der EP1379546 wird allgemein beschrieben, dass Peptide aus D-Aminosäuren an Amyloid-beta-Peptide anbinden können.

Molekulare Sonden, die dem Patienten intravenös injiziert werden und die nach der Passage über die Blut-Hirn-Schranke an Aß-Oligomere und Plaques binden, könnten mittels bildgebender Methoden sichtbar gemacht werden und somit eine frühere Diagnose der Alzheimerschen Demenz ermöglichen.

Es gibt bisher auch keinerlei Sonden für das in-vivo-Imaging-Verfahren, die speziell an Pyro-Glu-Aß-Spezies binden und diese sichtbar machen. Da Pyro-Glu-Aß-Oligomere in der Krankheitsgeschichte eine so wichtige und frühe Rolle spielen, ist genau dieses wünschenswert.

### Aufgabe und Lösung der Erfindung

Aufgabe der Erfindung ist es neue Kandidaten für eine
A) Ursächliche Therapie von Morbus Alzheimer
   und zum
B) Nachweis der Alzheimerschen Demenz im Frühstadium der Entstehung bereit zu stellen.

Die Aufgabe wird durch das Peptid nach dem Hauptanspruch gelöst.

Gemäß der Erfindung lösen Peptide die Aufgabe der Erfindung, welche an amino-terminal verkürztes Aβ-Peptid binden, dessen freiliegender Glutaminsäure-Rest an der Position 3 in zyklisierter Pyroglutamatform (AβpE3-x) vorliegt.

Es wurde erkannt, dass derartige Peptide die Bildung von toxischen Aβ-Oligomeren oder Aggregaten unterbinden können. Die Peptide binden vorteilhaft an im Körper vorkommendes, trunkiertes Amyloid-β-Peptid, das heißt an AβpE3-x, insbesondere an die pE3-42-Spezies. Die Bindung des Peptids an die monomere Form verhindert die Bildung von toxischen Aggregaten. Die Bindung an die oligomere Form löst diese wieder in untoxische Monomere auf oder detoxifiziert diese durch Umbildung zu untoxischen Aggregaten. Im Ergebnis wird vorteilhaft eine Enttoxifizierung durch die Bindung Peptid-zu-trunkiertes Amyloid-β-Peptid (AβpE3-x) gewährleistet.

Weiterhin werden die Peptide vorteilhaft als Sonden für den Einsatz in bildgebenden Methoden, wie z. B. Positronen-Emissions-Tomographie (PET) oder die Einzelphotonen-Emissionscomputertomographie (SPECT) eingesetzt.

Die vorliegende Erfindung betrifft daher auch Sonden, enthaltend ein Peptid gemäß Hauptanspruch, die für Sonden in der Bildgebung eingesetzt werden und zur Identifizierung von Aβ-Oligomeren, insbesondere von trunkierten Aβ-Oligomeren oder trunkierten Aβ-Monomeren (Plaques) verwendet werden.

Solche Sonden sind von großer Bedeutung, da damit eine frühe Diagnose der Alzheimerschen Demenz möglich wird. Damit kann der Krankheit schon in einem sehr frühen Stadium entgegengewirkt werden.

Solche molekularen Sonden enthalten das erfindungsgemäße Peptid und können den Patienten z. B. intravenös injiziert werden. Weitere Bestandteile der Sonden können sein: Farbstoffe, Fluoreszenzfarbstoffe, radioaktive Isotope (für z. B. PET), Gadolinium (für MRI) und/oder Bestandteile, die für Sonden in der Bildgebung eingesetzt werden. Nach der Passage über die Bluthirnschranke können die Sonden an die Aβ-Oligomere und/oder Plaques binden. Die so markierten, trunkierten Aβ-Oligomere und/oder Plaques aus trunkierten Aβ-Oligomeren können mittels bildgebender Verfahren wie z. B. SPECT, PET, CT, MRT, Protonen-MR-Spektroskopie usw. sichtbar gemacht werden und zwar auch in vivo.

Die erfindungsgemäßen Peptide können zur Verhinderung der Vermehrung von besonders toxischem, trunkierten Aβ-Oligomeren (AβpE3-x) verwendet werden.

Die erfindungsgemäßen Peptide können außerdem auch zur Bildung von nichttoxischen Peptid-Aβ-Oligomer-Komplexen verwendet werden.

Besonders vorteilhaft bestehen die erfindungsgemäßen Peptide überwiegend oder ausschließlich aus D-enantiomeren Aminosäuren.

Im Weiteren bedeutet der Begriff "überwiegend aus D-enantiomeren Aminosäuren", dass die einzusetzenden Monomere mindestens zu 60 %, bevorzugt zu 75 %, 80 %, besonders bevorzugt zu 85 %, 90%, 95%, insbesondere zu 96%, 97%, 98%, 99%, 100 % aus D-Aminosäuren aufgebaut sind.

Die D-enantiomeren Peptide sind vorteilhaft synthetisch herstellbar und kommen in der Regel in der Natur nicht vor. Sie verhalten sich zu L-enantiomeren Peptiden wie Bild und Spiegelbild. Sie sind durch Spiegelbild-Phagendisplaytechnik erhältlich.

Im Körper vorkommende Enzyme, wie z. B. Proteasen, oder Proteine des Immunsystems, erkennen vorteilhaft die D-enantiomeren Peptide nicht. Daher sind sie im Vergleich zu den L-enantiomeren Peptiden in vivo resistenter gegenüber Proteasen und lösen, wenn überhaupt, eine nur schwache Immunantwort hervor. Dies reduziert vorteilhaft die Gefahr von Nebenwirkungen signifikant.

In einer Spieglbild-Phagendisplayselektion wurden insgesamt vier neue D-enantiomere Peptide selektioniert, die vornehmlich an trunkiertes AβpE3-x binden.

Vier verschiedene dodekamere D-Peptide (Einbuchstabencode) wurden selektioniert und als neue Kandidaten bei der Lösung der Aufgabe identifiziert.

| | |
|---|---|
| D7 | HTRFEYYVYHMS gemäß SEQ ID NO:1 |
| D6 | AGERLKFIDEHV gemäß SEQ ID NO:2 |
| D4 | KMEHPNHPPPQR gemäß SEQ ID NO:3 und |
| D5 | NGAPNKIPRDRE gemäß SEQ ID NO:4. |

Diese wurden aus den selektierten spiegelbildlichen L-Peptiden abgeleitet. D4 und D5 hatten Aggregationskeim-frei präpariertes AβpE3-x als Selektionsziel, D6 und D7 jeweils niedermolekulare oder hochmolekulare AβpE3-x-Aggregate.

Die Strukturen nach einer der Sequenzen mit der SEQ ID NO:1, SEQ ID NO:2, SEQ ID NO:3 oder SEQ ID NO:4 sind besonders vorteilhaft neue Kandidaten für therapeutische Mittel zur Behandlung der Alzheimerschen Demenz.

Besonders vorteilhaft sind die Strukturen auch neue Kandidaten zum Nachweis der Alzheimerschen Demenz und der damit assoziierten Plaques.

Das Verfahren zum Nachweis von Plaques, welche zumindest teilweise aus amino-terminal verkürzten Aβ-Peptiden (AβpE3-x) bestehen, deren freiliegender Glutaminsäure-Rest an der Position 3 in zyklisierter Pyroglutamatform vorliegt, erfolgt mit den Schritten:
- in Kontakt bringen der Plaques *ex vivo* mit einem markierten Peptid oder einer Sonde, welches an das amino-terminal verkürzte Aβ-Peptid bindet,
- Nachweis der Plaques durch ein bildgebendes Verfahren.

Das Verfahren sieht die Auswertung der Bilder außerhalb des Körpers durch Vergleich mit Bildern von gesunden Probanden vor. Vorteilhaft wird dadurch der Nachweis von Plaques auch in vivo ermöglicht.

Besonders vorteilhaft werden Peptide zum Einsatz gebracht, welche überwiegend oder ausschließlich aus D-enantiomeren Aminosäuren bestehen.

Das Verfahren ist in einer Ausgestaltung der Erfindung gekennzeichnet durch Wahl eines Peptids mit einer der Sequenzen mit der SEQ ID NO:1, SEQ ID NO:2, SEQ ID NO:3 oder SEQ ID NO:4.

### Ausführunasbeispiel:

Im Weiteren wird die Erfindung an Hand eines Ausführungsbeispiels und der beigefügten Figur näher beschrieben, ohne dass es hierdurch zur Einschränkung der Erfindung kommen soll.

Die D-enantiomeren Peptide mit der SEQ ID NO:1-4 wurden wie folgt erhalten.

In einer Spieglbild-Phagendisplayselektion wurden insgesamt vier neue D-enantiomere Peptide selektioniert, die vornehmlich an trunkiertes AβpE3-x binden. Synthetisch hergestelltes AβpE3-x wurde als Zielmolekül in verschiedenen Spiegelbild-Phagendisplay-Selektionsprozessen eingesetzt. Die Methode des Phagendisplay ermöglicht es, aus einer sehr großen Bibliothek verschiedener Peptide diejenigen zu selektionieren, die an ein bestimmtes Zielmolekül binden. Dafür werden die Peptide auf Bakteriophagen präsentiert. Spiegelbild-Phagendisplay ist eine Sonderform des Phagendisplay. Im Phagendisplay wird das L-enatiomere Zielprotein als Target in der Selektion eingesetzt. Die Phagen präsentieren eine Peptidbibliothek auf ihrer Oberfläche und die Phagen, die Bindepartner für das Zielmolekül ("Target") präsentieren, werden durch iterative Runden von Bindung, Waschen und Amplifikation angereichert. Im Spiegelbild-Phagendisplay wird das Spiegelbild des eigentlichen Targets eingesetzt. Durch Phagendisplay werden dann L-enantiomere Peptide selektiert, die an das Spiegelbild des Zielmoleküls binden. Das Spiegelbild des so identifizierten L-Peptides (identische Aminosäuresequenz aber alle Aminosäuren bestehen aus D-Enantiomeren) bindet dann an das eigentliche, ursprüngliche Zielmolekül. Die Selektionen im Spiegelbild-Phagendisplay wurden gegen AβpE3-x ohne Aggregationskeime, das heißt hauptsächlich Monomer, und in niedermolekularer sowie hochmolekularer Aggregatform durchgeführt.

Für die Präparation von hochmolekularen Aggregaten von Pyroglutamat-Aβ wurden AβpE3-38-Bio (Bio: Biotin, zur Immobilisierung der Peptide zur Selektion auf Streptavidin-Mikrotiterplatten) sowie AβpE3-40 zu jeweils 10 sowie 100 µM in Hexafluoroisopropanol (HFIP) verdünnt. Anschließend wurden beide Peptide in einem Verhältnis von 1:20 zusammen zu 15 µg aliquotiert. Zur Präparation von hochmolekularen Aggregaten wurde der Peptidfilm in 5 µl Dimethylsulfoxid angelöst und in 95 µl physiologischem Puffer PBS (140 mM NaCl; 2.7 mM KCl; 10 mM Na₂HPO₄, pH 7,4) aufgenommen. Anschließend erfolgte die Inkubation bei 37°C und 500 rpm für 3 Tage, gefolgt von einer 1-stündigen Immobilisierung. Für die Präparation von niedermolekularen Aggregaten wurden ebenfalls AβpE3-38-Bio und AβpE3-40 eingesetzt. Zunächst wurde 871 ng AβpE3-38-Bio als Peptidfilm in NaOH angelöst und in Natriumphosphat-Puffer pH 7,4 (NaPi) aufgenommen und zu 87 ng pro Well aliquotiert. Nach einer 20-minütigen Immobilisierung wurde die Lösung abgenommen und verworfen. Anschließend wurde 871 ng AβpE3-40 ebenfalls in NaOH angelöst und in 250 µl NaPi aufgenommen, zu dem prä-immobilisierten Well gegeben und für weitere 2 Stunden immobilisiert. Für die Präparation von Peptiden ohne Aggregationskeime wurde die 1 mM Stammlösung von AβpE3-38-Bio in HFIP zu 1 µM weiter verdünnt und zu je 871 ng aliquotiert.

Nach dem Abdampfen von HFIP über Nacht wurde der Peptidfilm in 53 µl NaOH angelöst, 947 µl NaPi zugegeben und jeweils 100 µl mit 87 ng Aβ in die Wells pipettiert. Es folgte eine Immobilisierung für 30 Minuten.

In der Spieglbild-Phagendisplayselektion wurden neue D-enantiomere Peptide selektioniert, die vornehmlich an trunkiertes pEAβ3-x binden sollten. Die Selektionen im Spiegelbild-Phagendisplay wurden gegen AβpE3-x ohne Aggregationskeime, das heißt hauptsächlich Monomer, und in niedermolekularer sowie hochmolekularer Aggregatform durchgeführt. Vier verschiedene dodekamere D-Peptide D4 (KMEHPNHPPPQR gemäß SEQ ID NO:3), D5 (NGAPNKIPRDRE gemäß SEQ ID NO:4), D6 (AGERLKFIDEHV gemäß SEQ ID NO:2) und D7 (HTRFEYYVYHMS gemäß SEQ ID NO:1) wurden aus den selektierten spiegelbildlichen L-Peptiden abgeleitet. D4 und D5 hatten Aggregationskeim-frei präpariertes AβpE3-x als Selektionsziel, D6 und D7 jeweils niedermolekulare oder hochmolekulare AβpE3-x-Aggregate.

Die Bindeeigenschaften der vorliegenden Peptide an Aß-Plaques wurden untersucht. Für die Färbung von frontalen Hirnschnitten wurden Präparate transgener Tg2576-Mäuse im Alter von 10 bis 11 Monaten verwendet, die aufgrund des humanen APP-Gens mit der schwedischen Mutation eine erhöhte Aβ1-42 Expression und Plaquebildung aufweisen. In diesen Mäusen konnte auch AβpE3-x als Plaquebestandteil nachgewiesen werden.

Für die Versuche wurden die Peptide durch einen kommerziellen Anbieter synthetisch hergestellt und markiert. Anschließend wurde ex-vivo untersucht, welche Bereiche der Plaques vornehmlich gebunden werden. Dabei ist davon auszugehen, dass Aß-Plaques aus verschiedenen Aβ-Spezies, wie z. B. Aβ1-40, 1-42 und AβpE3-x bestehen.

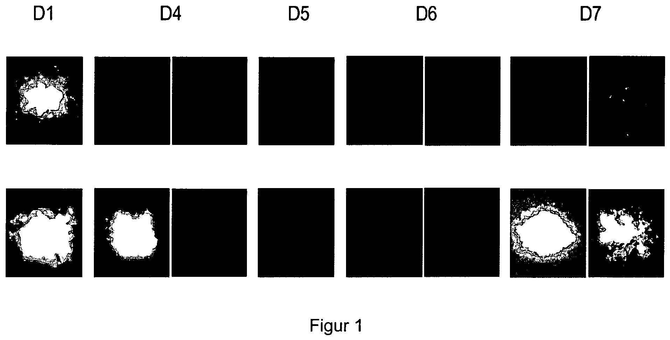

Die Ergebnisse sind in der Figur 1 dargestellt.
- Figur 1:: Histologische Färbung von Plaques im Gehirn (Kortex) von transgenen Alzheimermäusen (Tg2576), welche das humane Aβ**-**Vorläufer-Protein exprimieren, mit den D-enantiomeren Peptiden D4, D5, D6 und D7.

Alle Peptide sind zur Detektion mittels Fluoreszenzmikroskop mit einem Fluoresceinisothiocyanat (FITC) verknüpft. Als Positivkontrolle wurde das D1-FITC-Peptid, welches Aß-Plaques (speziell Aβ 1-42) bindet (Wiesehan et al., ChemBioChem 2003, 4, 748-753; Van Groen et al., ChemMedChem 2009, 4, 276-282), verwendet.

Als weitere Kontrolle wurden im Anschluss Kongorot-Färbungen derselben Proben durchgeführt, die zur Verifizierung der Plaquestrukturen dienten (jeweilige untere Abbildung). Es ist davon auszugehen, dass die Plaques aus verschiedenen Aβ-Isoformen (Aβ1-40, 1-42, AβpE3-x) zusammengesetzt sind. Schnitte von Wildtyp-Tieren ohne Plaquebildung wurden ebenfalls behandelt. In diesen Proben konnte keine Kongorot- sowie Fluoreszein-Fluoreszenz detektiert werden (Daten nicht gezeigt).

In den Gehirnschnitten, die mit D4, D5 und D6 behandelt wurden, wurden nur Plaques mit einer relativ dichten Struktur, bestehend aus Aβ-Fibrillen, gefärbt, keine kleinen und diffusen Plaques, bestehend aus amorphen Aβ-Aggregaten. Des Weiteren wurden ringförmige Strukturen um den inneren Kern der Plaques stärker gefärbt. D7 färbte ebenfalls nur dichte Strukturen, aber abweichend zu den anderen Peptiden den inneren Kern der Plaques. Dies deutet darauf hin, dass in den Plaques möglicherweise eine spezifische Verteilung der verschiedenen Aβ-Isoformen von den D-Peptiden erkannt wurde.

Die selektierten D-Peptide D4, D5, D6 und D7 sind somit erfindungsgemäß interessante Kandidaten für die Entwicklung von biomolekularen Plaquesonden und deren Verwendung in diagnostischen Nachweisverfahren. Die selektive und möglicherweise spezifische Affinität für unterschiedliche Plaquestrukturen und Aβ-Isoformen könnte die Diagnose der Alzheimerschen Demenz in bildgebenden Verfahren unterstützen und der Abgrenzung von anderen Amyloid assoziierten neurodegenerativen Krankheiten dienen. Eine Nutzung der Peptide für therapeutische Zwecke ist gegeben. Bisher sind keine Peptide bekannt, die in einer Selektion gegen pEAβ generiert wurden und die diagnostisch und therapeutisch genutzt werden könnten.

## Patentansprüche

1. Peptid, welches an amino-terminal verkürztes Aβ-Peptid bindet, dessen freiliegender Glutaminsäure-Rest an der Position 3 in zyklisierter Pyroglutamatform vorliegt
**gekennzeichnet durch**
ein ausschließlich oder mindestens zu 60% aus D-enantiomeren Aminosäuren bestehendes Peptid **gekennzeichnet durch** eine Struktur nach einer der Sequenzen mit der SEQ ID No:1, SEQ ID No:2, SEQ ID No:3 oder SEQ ID No:4.

2. Peptid nach Anspruch 1, zur Verwendung in der Medizin.

3. Sonde, enthaltend ein Peptid nach einem der Ansprüche 1 bis 2 sowie Bestandteile, die für Sonden in der Bildgebung eingesetzt werden.

4. Verfahren zum Nachweis von Plaques, welche zumindest teilweise aus amino-terminal verkürzten Aβ-Peptiden bestehen, deren freiliegender Glutaminsäure-Rest an der Position 3 in zyklisierter Pyroglutamatform vorliegt mit den Schritten:
- in Kontakt bringen der Plaques ex-vivo mit einem markierten Peptid oder einer Sonde, welches an das amino-terminal verkürzte Aβ-Peptid bindet, wobei ein mindestens zu 60% oder ausschließlich aus D-enantiomeren Aminosäuren bestehendes Peptid, **gekennzeichnet durch** eine Struktur nach einer der Sequenzen mit der SEQ ID No:1, SEQ ID No:2, SEQ ID No:3 oder SEQ ID No:4, gewählt wird,
- Nachweis der Plaques durch ein bildgebendes Verfahren.

## Claims

1. A peptide which binds to an amino-terminal truncated Aβ-peptide, the exposed glutamine acid residue of which is present at position 3 in cyclized pyroglutamate form,
**characterized by**
a peptide which consists exclusively or at least up to 60% of D-enantiomeric amino acids, **characterized by** a structure according to one of the sequences having the SEQ ID No:1, SEQ ID No:2, SEQ ID No:3 or SEQ ID No:4.

2. A peptide according to claim 1, for use in medicine.

3. A probe, containing a peptide according to any one of claims 1 to 2 as well as components which are used for probes in imaging.

4. A method for detecting plaques which at least partially consist of amino-terminal truncated Aβ-peptides, the exposed glutamine acid residue of which is present at position 3 in cyclized pyroglutamate form, with the steps:
- bringing the plaques ex-vivo into contact with a marked peptide or a probe, which peptide binds to the amino-terminal truncated Aβ-peptide, wherein a peptide, which consists of at least up to 60% or exclusively of D-enantiomeric amino acids, **characterized by** a structure according to one of the sequences having the SEQ ID No:1, SEQ ID No:2, SEQ ID No:3 or SEQ ID No:4, is selected,
- detecting the plaques by an imaging method.

## Revendications

1. Peptide qui fixe un peptide Aβ raccourci dans la partie terminale amino , dont le reste acide glutamique libre à la position 3 se présente sous la forme pyroglutamate cyclisé , **caractérisé par**
un peptide constitué exclusivement ou au moins pour 60 % d 'acides aminés énantiomères D , **caractérisé par** une structure suivant l'une des séquences ayant l'identification de séquence n°1 , l'identification de séquence n°2 , l 'identification de séquence n°3 ou l'identification de séquence n°4 .

2. Peptide suivant la revendication 1 ,
à utiliser en médecine .

3. Sonde contenant un peptide suivant l'une des revendications 1 à 2 , ainsi que des constituants qui sont utilisés pour des sondes dans l'imagerie .

4. Procédé pour déceler des plaques constituées au moins en partie de peptides Aβ raccourcis dans la partie terminale amino , dont le reste acide glutamique libre en la position 3 se présente sous la forme pyroglutamate cyclisé , comprenant les stades :
- on met les plaques ex-vivo en contact avec un peptide marqué ou une sonde qui fixe le peptide Aβ raccourci dans la partie terminale amino , dans lequel on choisit un peptide constitué pour au moins 60 % ou exclusivement d'acides aminés énantiomères D **caractérisé par** une structure suivant l'une des séquences ayant l'identification de séquence n°1 , l 'identification de séquence n°2 , l'identification de séquence n°3 ou l'identification de séquence n°4 ,
- on décèle les plaques par un procédé d'imagerie .
